# EUROPEAN PATENT APPLICATION

(11) **EP 4 843 509 A2**
(43) Date of publication of application: **25.11.2026**
(21) Application number: 26179608.0
(22) Date of filing: 19.05.2026
(51) Int. Cl.: A61B 17/00, A61B 90/90, A61B 17/072, A61B 90/00

(54) **TECHNOLOGIES FOR END EFFECTOR SLED DETECTION USING MAGNETIC FIELD DISTORTION FOR SURGICAL STAPLING INSTRUMENTS**

(30) Priority: 19.05.2025 US 202519212549
(71) Applicant: Cilag GmbH International, 6300 Zug (CH)
(72) Inventor: OVERMYER, Mark D, 6300 Zug (CH); HOLDMEYER, Seth D, 6300 Zug (CH); MOZLOOM, Joseph T, 6300 Zug (CH); LIU, Zhijun, 6300 Zug (CH); JONES, Daniel V, 6300 Zug (CH); PASKO, Kevin, 6300 Zug (CH); ULRICH, Daniel J, 6300 Zug (CH)
(74) Representative: Carpmaels & Ransford LLP

(57) **Abstract**

A method for controlling a surgical instrument includes measuring, by a controller, a magnetic field signature with a magnetic field sensor coupled to an end effector of the surgical instrument. The surgical instrument includes the end effector, the magnetic field sensor, and a permanent magnet coupled to the end effector. The method further includes detecting a deviation between the measured signature and a predetermined signature that is associated with a stapler cartridge being present in the end effector, with a sled of the cartridge positioned at a home position within the end effector. The method further includes determining whether a cartridge is present based on the detected deviation, and inhibiting firing of the surgical instrument in response to determining that the cartridge is not present. Other embodiments are described and claimed.

## Description

### CROSS REFERENCE TO RELATED APPLICATIONS

The present application claims benefit of and priority to U.S. Non-Provisional Patent Application No. 19/212,549, filed May 19, 2025, with the United States Patent & Trademark Office, the contents of which are hereby incorporated by reference in its entirety.

### TECHNICAL FIELD

The present disclosure relates generally to surgical instruments and, more particularly, to surgical stapling and cutting instruments and associated staple cartridges for use with the surgical instruments to facilitate the cutting and stapling of patient tissue.

### BACKGROUND

Surgical procedures often involve the use of various surgical instruments to assist a surgeon in the performance of the corresponding procedure. Recently, minimally invasive surgical (MIS) instruments have gained preference over traditional "open" surgical devices as the use of MIS instruments can reduce post-operative recovery time and associated tissue scarring. Endoscopy and laparoscopy are common types of MIS procedures in which a tube or trocar is inserted into natural or surgically-made openings (e.g., incisions) of the patient, depending on the particular procedure. The tube or trocar is then used to facilitate introduction of associated surgical instruments into the patient's body (e.g., the patient's abdominal cavity) to treat internal tissue of the patient.

One type of surgical instrument usable in MIS procedures is a surgical stapler, sometimes referred to as an "endocutter." A typical surgical stapler includes an elongated shaft to facilitate use of a trocar and an end effector located at a distal end of the elongated shaft. The elongated shaft facilitates the use of the surgical stapler with a trocar to access the patient's anatomical environment (e.g., the patient's abdominal cavity) and may include one or more articulation joints to increase the usability of the surgical stapler in the anatomical environment. Similarly, the end effector may be attached to the elongated shaft via an articulation joint to allow the end effector to be positioned as desired by the surgeon. A typical end effector of a surgical stapler includes a pair of jaws movable between an open position and a closed position to facilitate the grasping of tissue within the jaws. One of the jaws is embodied as a cartridge jaw and is configured to receive a staple cartridge, and the other jaw is embodied as an anvil jaw configured to provide a surface on which the staples are formed. The end effector also includes a cutting element (typically referred to as a "knife"), which is translated distally along the end effector during a firing phase of the surgical stapler to transect tissue presently grasped by the end effector. As the knife advances along the end effector, staples contained in the staple cartridge are progressively ejected to thereby seal opposing sides of the transected tissue.

Surgical staplers may be embodied as independent, hand-held devices or configured to be coupled to a robotic surgery system. In hand-held embodiments, the surgical stapler may include a handle and an associated trigger, which is operable by a surgeon to control the end effector and the ejection of the staples from the staple cartridge. In robotic embodiments, the surgical stapler is configured to be mounted to a robotic arm of a robotic manipulator, which is controllable by the surgeon via a remote control console. For example, the control console may include various input devices that can be grasped and manipulated by the surgeon to cause movement and firing of the surgical stapler.

### SUMMARY

According to one aspect, a method for controlling a surgical instrument comprises measuring, by a controller coupled to the surgical instrument, a magnetic field signature with a magnetic field sensor coupled to an end effector of the surgical instrument, wherein a permanent magnet is coupled to the end effector; determining, by the controller, a deviation between the magnetic field signature and a first predetermined magnetic field signature, wherein the first predetermined magnetic field signature is associated with a cartridge present state of the surgical instrument, wherein the cartridge present state occurs when a cartridge is attached to the end effector and a sled of the cartridge is positioned at a home position within the end effector; determining, by the controller, whether the surgical instrument is in the cartridge present state based on the deviation; and inhibiting, by the controller, firing of the surgical instrument in response to determining that the surgical instrument is not in the cartridge present state.

In an embodiment, the sled of the cartridge comprises a ferromagnetic material. In an embodiment, measuring the magnetic field signature comprises measuring the magnetic field signature with a three-dimensional Hall effect sensor. In an embodiment, measuring the magnetic field signature comprises measuring the magnetic field signature with a plurality of reed switches.

In an embodiment, measuring the magnetic field signature comprises measuring a first magnetic field value with the magnetic field sensor; activating an electromagnet coupled to the end effector; and measuring a second magnetic field value with the magnetic field sensor while the electromagnet is activated, wherein the magnetic field signature comprises the first magnetic field value and the second magnetic field value. In an embodiment, the electromagnet comprises a near-field communication antenna of the end effector.

In an embodiment, determining the deviation between the magnetic field signature and the first predetermined magnetic field signature comprises comparing a first plurality of magnetic field values of the magnetic field signature to a corresponding second plurality of magnetic field values of the first predetermined magnetic field signature; and determining whether the surgical instrument is in the cartridge present state comprises determining whether any value of the first plurality of magnetic field values differs by more than a predetermined threshold amount from the corresponding value of the second plurality of magnetic field values.

In an embodiment, determining the deviation between the magnetic field signature and the first predetermined magnetic field signature comprises providing the magnetic field signature to a decision model (e.g., a pretrained machine learning model); and determining whether the surgical instrument is in the cartridge present state comprises recognizing the cartridge present state with the decision model.

In an embodiment, the method further comprises determining, by the controller, a type of cartridge coupled to the end effector based on the magnetic field signature. In an embodiment, determining the type of the cartridge comprises comparing the magnetic field signature to a library of predetermined magnetic field signatures.

In an embodiment, the method further comprises providing, by the controller, diagnostic data based on the magnetic field signature in response to determining that the surgical instrument is not in the cartridge present state. In an embodiment, the method further comprises permitting, by the controller, firing of the surgical instrument in response to determining that the surgical instrument is in the cartridge present state.

According to another aspect, a surgical instrument includes an end effector, a permanent magnet, and a magnetic field sensor. The end effector includes an elongated jaw that extends to a distal end. The jaw includes a first side wall, a second side wall, and a floor coupled between the first side wall and the second side wall. The first side wall, the second side wall, and the floor cooperate to define a channel extending to the distal end. The permanent magnet is coupled to the elongated jaw. The magnetic field sensor is coupled to the elongated jaw and configured to measure a magnetic field signature associated with the permanent magnet.

In an embodiment, the surgical instrument further includes an elongated staple cartridge positioned in the channel of the jaw. The staple cartridge includes a sled moveable from a proximal position to a distal position. The magnetic field signature associated with the permanent magnet changes when the sled moves between the proximal position and the distal position. In an embodiment, the sled comprises a ferromagnetic material. In an embodiment, the sled includes an outer fin that extends upward. The outer fin includes a fin extension that extends proximally from the outer fin. The fin extension redirects magnetic field from the permanent magnet when the sled is in the proximal position. In an embodiment, the magnetic field sensor comprises an array of a plurality of magnetic field sensors. The fin extension prevents the magnetic field from the permanent magnet from reaching a predetermined selection of the plurality of magnetic field sensors when the sled is in the proximal position.

In an embodiment, the sled includes a lateral extension that extends toward the magnetic field sensor, the end effector includes a magnetic driver, and when the sled moves from the proximal position to the distal position, the lateral extension forces the magnetic driver toward the magnetic field sensor, whereby the magnetic field signature associated with the permanent magnet changes.

In an embodiment, the permanent magnet is coupled to the first side wall and the magnetic field sensor is coupled to the first side wall adjacent to the permanent magnet. In an embodiment, the permanent magnet is coupled to the first side wall and the magnetic field sensor is coupled to the second side wall across the channel from the permanent magnet.

In an embodiment, the surgical instrument further includes a near-field antenna coupled to the end effector. The near-field antenna is configured to generate a magnetic field when energized. The magnetic field sensor is further configured to measure the magnetic field generated by the near-field antenna when energized.

In an embodiment, the surgical instrument further includes a board support coupled to the elongated jaw. The board support includes a first arm and a second arm coupled by a bend. The surgical instrument further includes a circuit board coupled to the first arm of the board support. The circuit board includes the magnetic field sensor. The permanent magnet is coupled to the second arm of the board support, and the permanent magnet is positioned across the channel from the magnetic field sensor.

### BRIEF DESCRIPTION OF THE DRAWINGS

The detailed description particularly refers to the following figures, in which:
FIG. 1 is a perspective view of an embodiment of a surgical stapler configured for use with a robotic surgical system;
FIG. 2 is a perspective view of an embodiment of an end effector of the surgical stapler of FIG. 1;
FIG. 3 is an exploded perspective view of and embodiment of a distal end of the surgical stapler of FIG. 1, including the end effector of FIG. 2;
FIG. 4 is a perspective view of an embodiment of a I-beam of the end effector of FIG. 2, which includes a knife;
FIG. 5 is a cross-sectional view of the end effector of FIG. 1 in a closed state and showing the I-beam of FIG. 4 received in corresponding channels of the end effector of FIG. 2 with the knife of the I-beam extending between the corresponding channels;
FIG. 6 is a perspective view of an embodiment of a stapler cartridge that may be inserted into the end effector of FIG. 2;
FIG. 7 is an exploded, perspective view of the stapler cartridge of FIG. 6;
FIG. 8 is a simplified block diagram of an embodiment of a robotic surgical system including a control console and a robotic manipulator, with which the surgical stapler of FIG. 1 may be used;
FIG 9 is a diagrammatic view of an embodiment of a tool interface located on a bottom side of a drive housing of the surgical stapler of FIG. 1 being coupled to an arm interface located on a robotic arm of the robotic manipulator of the robotic surgical system of FIG. 8;
FIG. 10 is a perspective view of an embodiment of a hand-held surgical stapler including the end effector of FIG. 2;
FIG. 11 is a schematic view of an end effector of the system of FIGS. 1-10 including one or more magnetic field sensors;
FIG. 12 is a simplified flow diagram of at least one embodiment of a method for non-mechanical firing lockout using magnetic field detection that may be executed by a controller of the surgical stapler of FIGS. 1-10 in connection with the end effector of FIG. 11;
FIGS. 13-16 are perspective views of multiple potential embodiments of sleds that may be included in a staple cartridge of FIGS. 11-12;
FIG. 17 is a schematic view of another embodiment of an end effector of the system of FIGS. 1-10 including one or more magnetic field sensors that may be used in connection with the method of FIG. 12;
FIG. 18 is a perspective view of at least one embodiment of an electronics system of the end effector of FIGS. 1-10 that includes magnetic field sensors and that may be used in connection with the method of FIG. 12; and
FIGS. 19-20 are views of an end effector of FIGS. 1-10 including the electronics system of FIG. 18, which may be used with the method of FIG. 12.

### DETAILED DESCRIPTION OF THE DRAWINGS

While the concepts of the present disclosure are susceptible to various modifications and alternative forms, specific illustrative embodiments thereof have been shown by way of example in the drawings and will herein be described in detail. It should be understood, however, that there is no intent to limit the concepts of the present disclosure to the particular forms disclosed, but on the contrary, the intention is to cover all modifications, equivalents, and alternatives falling within the spirit and scope of the invention as defined by the appended claims.

Terms representing anatomical references, such as anterior, posterior, medial, lateral, superior, inferior, distal, proximal, et cetera, may be used throughout the specification in reference to the surgical instruments described herein as well as in reference to the patient's natural anatomy. Such terms have well-understood meanings in both the study of anatomy and the field of surgery. Use of such anatomical reference terms in the written description and claims is intended to be consistent with their well-understood meanings unless noted otherwise.

References in the specification to "one embodiment," "an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to effect such feature, structure, or characteristic in connection with other embodiments whether or not explicitly described. Additionally, it should be appreciated that items included in a list in the form of "at least one A, B, and C" can mean (A); (B); (C); (A and B); (A and C); (B and C); or (A, B, and C). Similarly, items listed in the form of "at least one of A, B, or C" can mean (A); (B); (C); (A and B); (A and C); (B and C); or (A, B, and C).

Portions of the disclosed embodiments may be implemented, in some cases, in hardware, firmware, software, or any combination thereof. The disclosed embodiments may also be implemented as instructions carried by or stored on a transitory or non-transitory machine-readable (e.g., computer-readable) storage medium, which may be read and executed by one or more processors. A machine-readable storage medium may be embodied as any storage device, mechanism, or other physical structure for storing or transmitting information in a form readable by a machine (e.g., a volatile or non-volatile memory, a media disc, or other media device).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, may not be included or may be combined with other features.

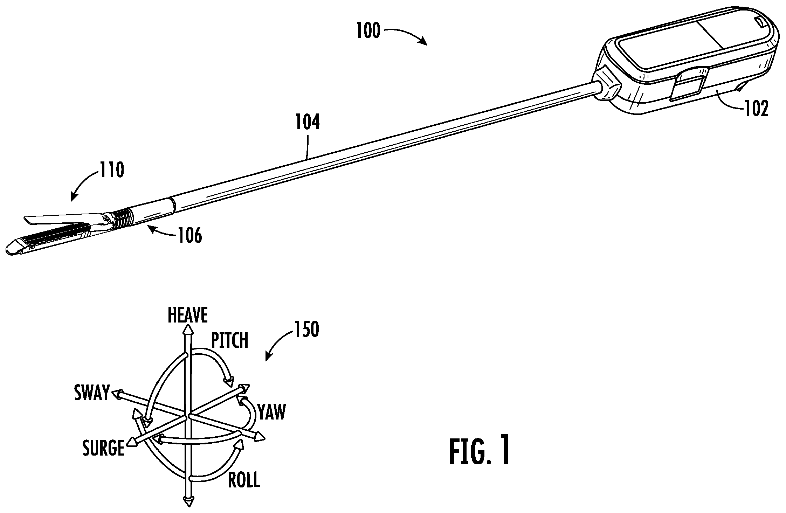

Referring now to FIG. 1, in an illustrative embodiment, a surgical stapler or "endocutter" 100 is configured for use in minimally invasive surgery (MIS) procedures including, but not limited to, endoscopic and laparoscopic procedures. The surgical stapler 100 is configured to contemporaneously transect and staple tissue during the performance of such surgical procedures. Although the concepts presented below are described in regard to a surgical stapler, it should be appreciated that the presented concepts may be applicable to other types of surgical instruments configured to perform different surgical functions including, but not limited to, surgical scissors, tissue graspers, energy-based surgical instruments, clip appliers, needle drivers, and/or other surgical instruments.

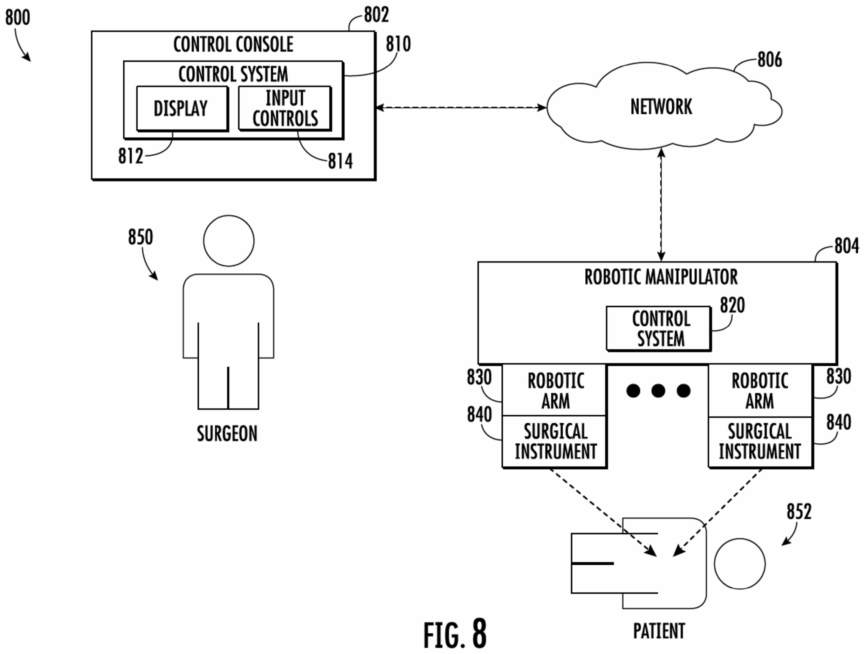

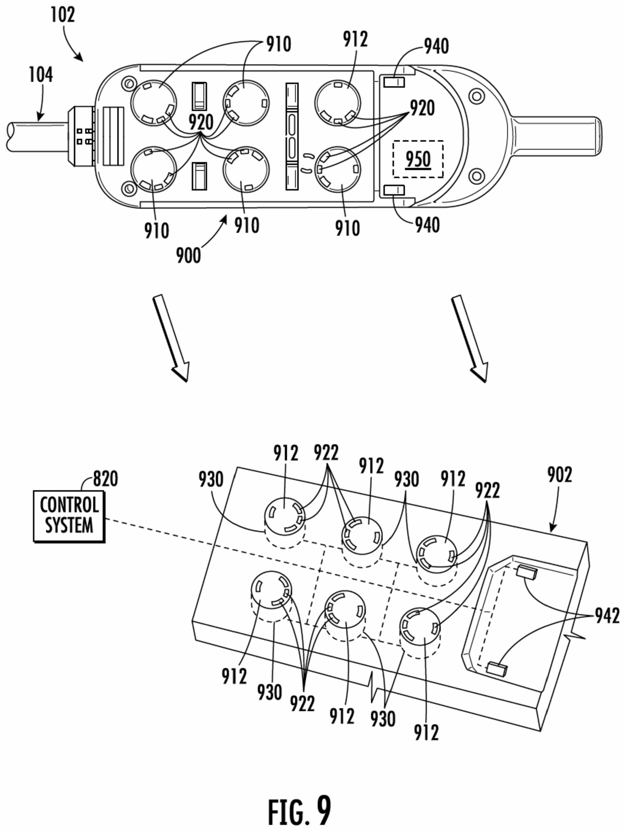

The illustrative surgical stapler 100 includes a drive housing 102, an elongated shaft 104 that extends distally away from the drive housing 102, and an end effector 110 located at a distal end of the elongated shaft 104 opposite the drive housing 102. As discussed in more detail below, the surgical stapler 100 is embodied as a robotic surgical stapler and is configured to be used with a corresponding robotic surgical system. As such, the drive housing 102 includes coupling features that that facilitate the mounting of the drive housing 102 to a robotic arm of a robotic manipulator of the robotic surgery system as discussed below in regard to FIGS. 8 and 9. The mounting of the drive housing 102 to the robotic arm allows the robotic manipulator to control various functions of the surgical stapler 100, including the movement and "firing" of the end effector 110, as discussed in more detail below.

The elongated shaft 104 is sized and configured for use in minimally invasive surgery procedures. For example, the elongated shaft 104 has a length and diameter that is sized to allow the elongated shaft 104 to be inserted into a trocar or similar surgical tube to allow positioning of the end effector 110 into the patient's anatomical environment (e.g., the patient's abdominal cavity). Illustratively, the distal end of the elongated shaft 104 is coupled to the end effector 110 via an articulable joint 106, which allows the end effector 110 to be moved to different orientations and/or positions relative to the elongated shaft 104.

In the illustrative embodiment, the articulable joint 106 provides six degrees of freedom to the end effector 110. For example, as indicated by coordinate frame 150, the degrees of freedom of the end effector 110 may include three translational degrees (i.e., surge, heave, and sway) and three rotational degrees (i.e., roll, pitch, and yaw). The "surge" degree of freedom refers to forward and backward translational movement of the end effector 110 relative to the elongated shaft 104, the "heave" degree of freedom refers to upward and downward translational movement of the end effector 110 relative to the elongated shaft 104, and the "sway" degree of freedom refers to left and right translational movement of the end effector 110 relative to the elongated shaft 104. The "roll" degree of freedom refers to rotation of the end effector 110 along a longitudinal axis defined by the end effector 110, the "pitch" degree of freedom refers to upward and downward tilting of the end effector 110 relative to the elongated shaft 104, and the "yaw" degree of freedom refers to leftward or rightward turning of the distal end of the end effector 110 relative to the elongated shaft 104.

The elongated shaft 104 also houses a portion of an actuation system (not shown) to control the movement and activation (e.g., the "firing") of the end effector 110. The actuation system may include various articulation cables, push rods, firing rods, and/or other devices, which extend through the elongated shaft 104 from mechanisms located in the drive housing 102 to components of the articulable joint 106 and/or the end effector 110. In this way, the drive housing 102 is configured to control the movement and activation (i.e., the "firing") of the end effector 110.

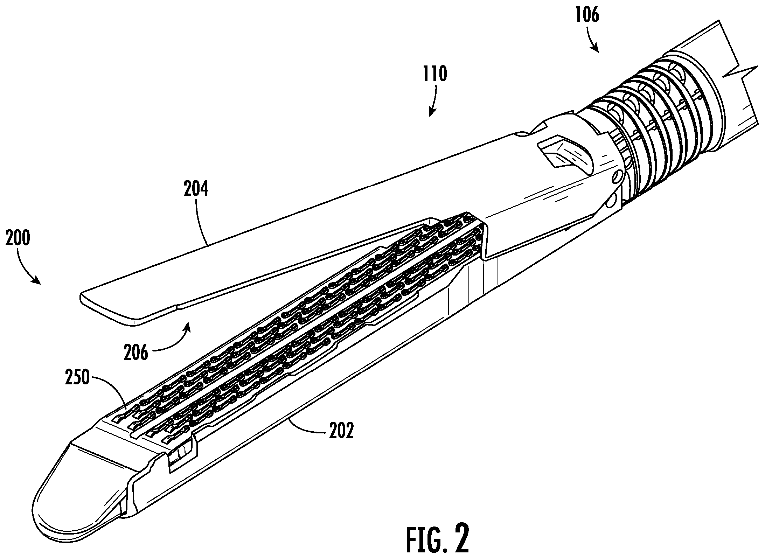

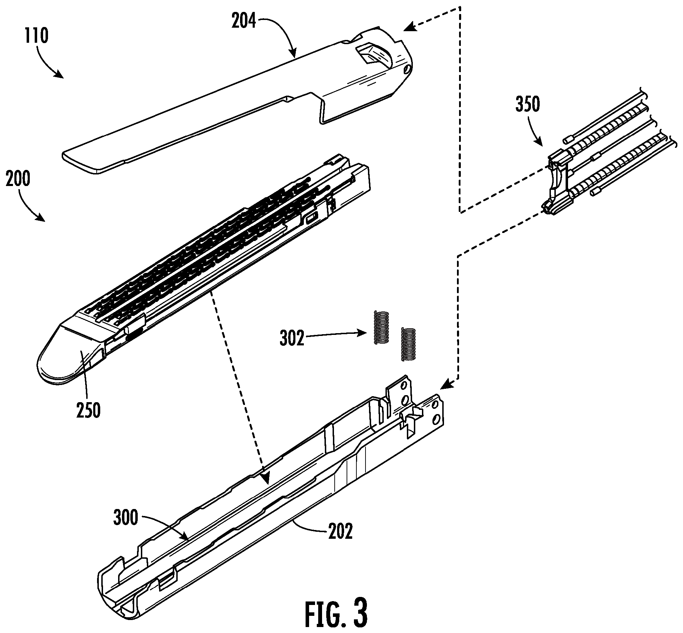

Referring now to FIG. 2, the end effector 110 includes a jaw assembly 200, which illustratively includes a pair of jaws 202, 204 that oppose each other. The jaw 202 is illustratively embodied as a "cartridge" jaw and includes a channel 300 (see FIG. 3) configured to receive a staple cartridge 250. The jaw 204 is illustratively embodied as an "anvil" jaw and includes a bottom surface 206 having forming pockets configured to deform staples ejected from the staple cartridge 250.

The jaw assembly 200 is movable between an open state in which the anvil jaw 204 is positioned away from the cartridge jaw 202 and a closed state in which the anvil jaw 204 is positioned near or otherwise contacts the cartridge jaw 202. Illustratively, the anvil jaw 204 is configured to move toward and away from the cartridge jaw 202. However, in other embodiments, the cartridge jaw 202 may be configured to move relative to the anvil jaw 204 or both jaws 202, 204 may be configured to move toward or away from each other. Additionally, it should be appreciated that the open state may correspond to a degree of openness that is less than a fully opened position of the jaw assembly 200 and the closed state may correspond to a degree of closeness that is less than a fully closed position. That is, the closed state may, for example correspond to a minimal distance between the distal ends of the cartridge jaw 202 and the anvil jaw 204 and the open state may correspond to a maximum distance between the distal ends of the jaws 202, 204. However, in other embodiments, the open state may correspond to a fully opened position of the jaw assembly 200 and the closed state may correspond to a fully closed position of the jaw assembly 200.

As shown in FIG. 3, the jaw assembly 200 is biased in the open state by a pair of springs 302. That is, the springs 302 bias the anvil jaw 204 away from the cartridge jaw 202. However, actuation of the jaw assembly 200 overcomes the biasing force of the springs 302 to move the jaw assembly 200 from the open state to the closed state. When in the closed state, the end effector 110 can be "fired" to effect the cutting and stapling of tissue held within the jaw assembly 200

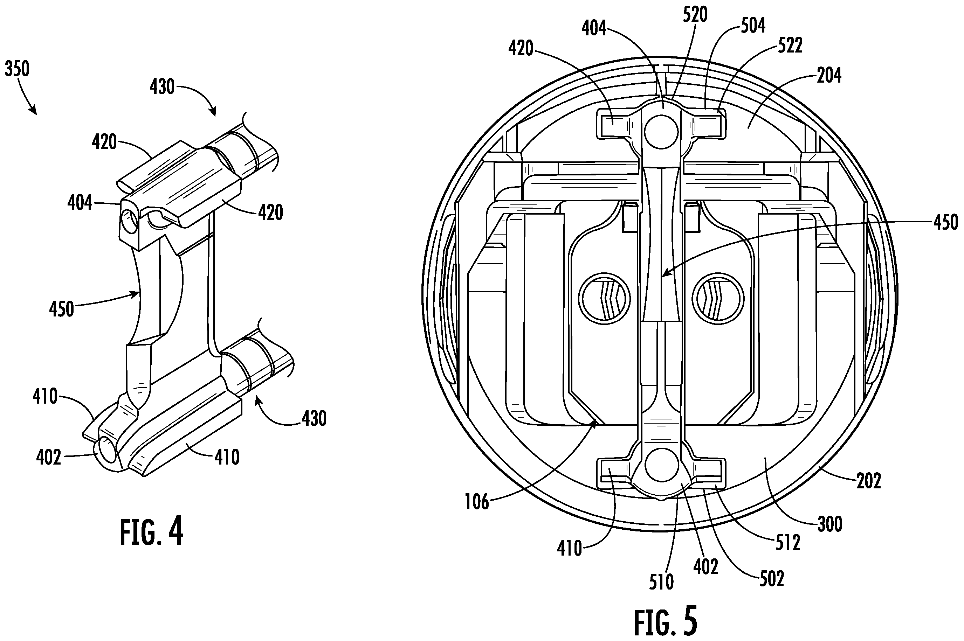

The end effector 110 also includes a I-beam 350, which is configured to translate forward during the firing of the end effector 110 from the proximal end of the jaws 202 toward the distal end of the jaws 202, 204. To do so, portions of the I-beam 350 are received in corresponding channels of the cartridge jaw 202 and the anvil jaw 204 as discussed in more detail below in regard to FIGS. 4 and 5. When the end effector 110 is fired, the I-beam 350 translates forward within the channels of the jaws 202, 204. The forward movement of the I-beam 350 may cause the anvil jaw 204 to move further downward toward or against the cartridge jaw 202 and clamp the jaw assembly in the closed state.

Referring now to FIGS. 4 and 5, the I-beam 350 includes a lower base 402, an upper base 404, and a knife 450 located between the upper and lower bases 402, 404. A pair of lower wings 410 extend laterally outward from the lower base 402. Similarly, a pair of upper wings 420 extend laterally outward from the upper base 404. A push rod 430 is coupled to each of the bases 402, 404 and to control mechanisms located in the drive housing 102. The control mechanisms of the drive housing 102 control movement of the push rods 430, which moves the I-beam 350 forward and backward within the jaw assembly 200.

When the end effector 110 is fired, the I-beam 350 moves within a lower I-beam channel 502 of the cartridge jaw 202 and an upper I-beam channel 504 of the anvil jaw 204 as shown in FIG. 5. The I-beam channel 502 of the cartridge jaw 202 includes a base channel 510 and a pair of wing channels 512, which are open to and in fluid communication with the base channel 510 and extend laterally outward therefrom. The lower base 402 of the I-beam 350 is received in and moves within the base channel 510 of the I-beam channel 502. Similarly, each lower wing 410 is received in and moves within a corresponding one of the wing channels 512. The I-beam channel 504 of the anvil jaw 204 also includes a base channel 520 and a pair of wing channels 522, which are open to and in fluid communication with the base channel 520 and extend laterally outward therefrom. The upper base 404 of the I-beam 350 is received in and moves within the base channel 520 of the I-beam channel 502. Similarly, each upper wing 420 is received in and moves within a corresponding one of the wing channels 522.

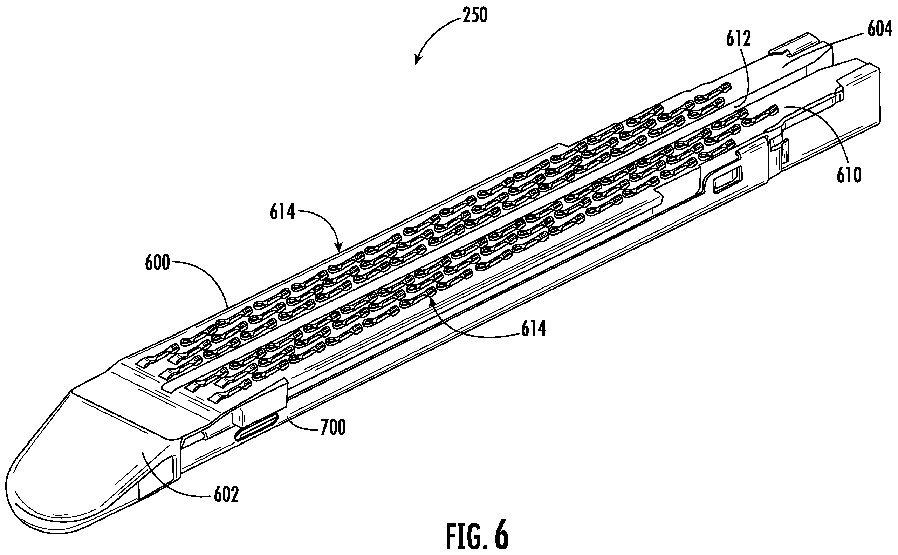

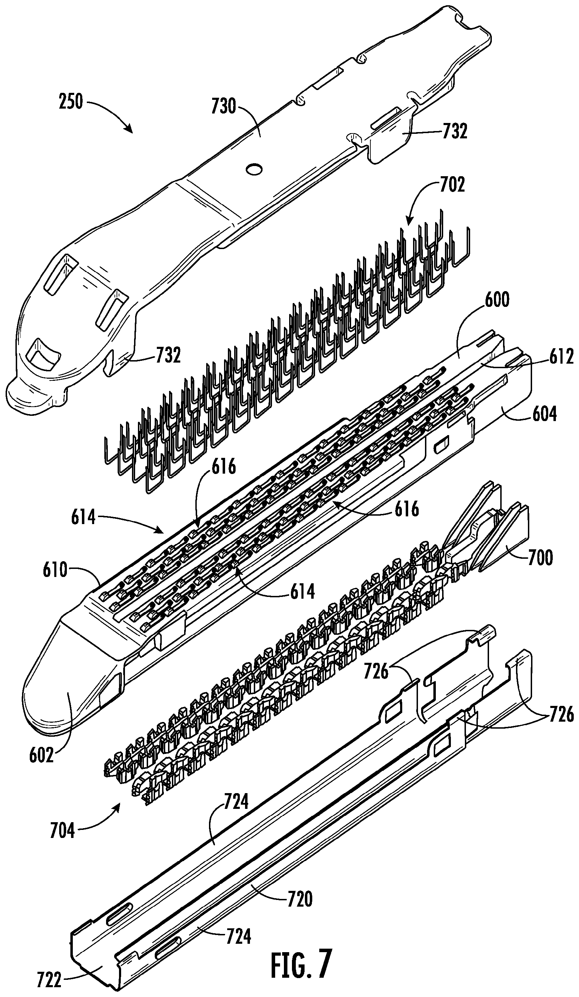

Referring now to FIGS. 6 and 7, the illustrative staple cartridge 250 includes a cartridge body 600 having a distal end 602 and a proximal end 604. The cartridge body 600 also includes a deck 610 that extends from the proximal end 604 to the distal end 602. The deck 610 includes a longitudinal knife slot 612 that is located centrally on the deck 610 and extends from the distal end 602 toward the proximal end 604 of the cartridge body 600. During a firing stroke of the surgical stapler 100 (i.e., when the end effector is "fired"), the I-beam 350 transversed along the longitudinal knife slot 612 with the knife 450 of the I-beam 350 protruding upwardly from the longitudinal knife slot 612 to facilitate the transection of tissue captured within in the end effector 110 and held between the staple cartridge 250 and the anvil jaw 204.

A set of staple cavities 614 are defined in the deck 610 on either side of the longitudinal knife slot 612. A staple 702 is positioned in each of the staple cavities 614 and is supported by a corresponding staple driver 704. The staple cartridge 250 also includes a staple sled 700, which is initially located toward the proximal end 604 of the cartridge body 600 and is pushed toward the distal end 602 by the I-beam 350 when the end effector 110 is fired. The staple sled 700 lifts each staple driver 704 when the sled 700 comes into contact with the corresponding staple driver 704, which ejects the staple 702 associated with the corresponding staple driver 704 from the corresponding staple cavity 614. In the illustrative embodiment, the deck 610 includes staple guides or projections 616 that project upwardly from the deck 610 around each staple cavity 614. The staple guides 616 are configured to guide or control the legs of the staples 702 as the staples 702 are being ejected from the staple cavities 614. Illustratively, the staple guides 616 are located around the distal and proximal ends of each staple cavity 614, but may completely surround each staple cavity 614 or be located on only one end of each staple cavity 614 in other embodiments. The staple guides 616 may also form a set of "teeth" and be configured to grasp tissue held within the jaw assembly 200 to restrict movement of the tissue. In other embodiments, the deck 610 of the cartridge body 600 maybe devoid of any staple guides 616.

The staple cartridge 250 also includes a pan 720, which is attached to the cartridge body 600 and is configured to retain the staple drivers 704 and associated staples 702 within the cartridge body 600. Illustratively, the pan 720 is formed from a metallic material and includes a floor 722 and a pair of sidewalls 724 that extend upwardly from the floor 722 and wrap onto the sides of the cartridge body 600. The pan 720 includes a set of attachment tabs 726 that are configured to secure the pan 720 to the cartridge body 600.

Prior to use, the staple cartridge 250 includes a cover 730 attached to the cartridge body 600. The cover 730 is configured to cover the deck 610 of the staple cartridge 250 and includes a set of attachment tabs 732 configured to secure the cover 730 to the cartridge body 600. To prepare the staple cartridge 250 for use in the end effector 110, the cover 730 is removed from the cartridge body 600.

Although not illustrated in FIGS. 6 and 7, the staple cartridge 250 may also includes one or more electronic circuits or devices configured to perform one or more associated functions. For example, such electronic circuitry may include processors, digital storage devices, communication circuitry, sensor circuitry, and/or other electrical components.

Referring now to FIG. 8, as discussed above, the illustrative surgical stapler 100 is configured for use with a corresponding robotic surgical system 800. The robotic surgical system 800 includes a control console 802 and a robotic manipulator 804, which communicate with each other over a communication network 806. Although only a single control console 802 and a single robotic manipulator 804 is shown in FIG. 8, it should be appreciated that the robotic surgical system 800 may include additional control consoles 802 and/or robotic manipulators 804 in other embodiments.

The control console 802 is usable by a surgeon 850 to control the operation of the robotic manipulator 804. To do so, the control console 802 includes a control system 810. Illustratively, the control system 810 includes a display 812 and one or more input controls 814. However, it should be appreciated that the control system 810 may include additional electrical components and devices, such as a processor, a memory, and a communication subsystem to enable communications of the components of the control system 810, which are not illustrated in FIG. 8 for clarity.

The display 812 may be embodied as any type of display device capable of generating images viewable by the surgeon 850. In use, the display 812 may display images related to the surgical procedure being performed via the robotic manipulator 804. The displayed images may be obtained from, for example, an endoscopic camera operated by the robotic manipulator 804. Additionally, the display 812 may display information, including data determined by the control console 802 and/or the robotic manipulator 804, related to the surgical procedure (e.g., positional data of the robotic manipulator 804).

The input controls 814 are usable by the surgeon 850 to control the functionality of the robotic manipulator 804. The input controls 814 may be embodied as any type of input device capable of receiving a corresponding input from the surgeon 850. For example, the input controls 814 may include physical controllers, such as joy sticks, hand-held actuator modules, exoskeletal gloves, and/or other input devices. The input controls 814 may also include input devices other than hand-controlled devices such as foot pedals, vison tracking modules, and/or the like. In many embodiments, the input controls 814 are movable in multiple degrees of freedom to control the positioning and operation of the robotic manipulator 804.

The robotic manipulator 804 also includes a control system 820 and one or more robotic arms 830 to which surgical instruments 840 (e.g., the surgical stapler 100) may be mounted. In some embodiments, the robotic manipulator 804 may be mounted to a transport cart, sometimes referred to as an "arm cart," that enables mobility of the robotic manipulator 804 and the associated robotic arms 830.

The robotic arms 830 may include various articulable linkages and associated motors, which are controllable by the control console 802 to move the corresponding robotic arm 830 and any associated surgical instrument 840 to a desired position. For example, by manipulating an input control 814 of the control console 802, the surgeon 850 may control the positioning of a corresponding robotic arm 830, as well as the functionality of the associated surgical instrument 840 (e.g., the firing of the surgical stapler 100).

The control system 820 may be embodied as any type of controller or control circuit capable of controlling the functionality of the robotic manipulator including, for example, the movement of the robotic arms 830 and the activation of the surgical instruments 840 based on control signals received from the control console 802. To do so, the control system 820 may include various electrical components, circuits, and/or devices, such as a processor, a memory, and a communication subsystem to enable communications of the components of the control system 820, which are not illustrated in FIG. 8 for clarity.

In use, the robotic manipulator 804 is positioned in close proximity to a patient 852 requiring surgery. The robotic manipulator 804 may be locked or mounted in place for the duration of the surgery. The surgeon 850 may then manipulate the input controls 814 to position one or more robotic arms 830 and associated surgical instruments 840 into a desired position. For example, the surgeon 850 may position a robotic arm 830 such that an associated surgical instrument 840 is inserted through a trocar or similar elongated passageway into the anatomical environment (e.g., the abdominal cavity of the patient 852). Once so positioned, some surgical instruments 840 (e.g., an endoscope) may be locked into position to avoid unintended repositioning.

The network 806 may be embodied as any type of wired and/or wireless network or set of communication links capable of facilitating communications between the control console 802 and the robotic manipulator 804. To do so, the network 806 enable such communications using any suitable data communication specification and/or protocol. As such, in some embodiments, the network 806 may include additional devices, such as additional computers, routers, stations, and/or switches, to facilitate such communications between the control console 802 and the robotic manipulator 804.

Referring now to FIG. 9, as discussed above, the surgical stapler 100 (and other surgical instruments 840) is configured to be mounted to a robotic arm 830 of the robotic manipulator 804. To facilitate such mounting, the drive housing 102 of the surgical stapler 100 includes a tool interface 900 located on a bottom side of the drive housing 102. The tool interface 900 is configured to mate with an arm interface 902 of the corresponding robotic arm 830 to couple the surgical stapler 100 to the robotic arm 830. The coupling of the surgical stapler 100 to the robotic arm 830 may be further facilitated via various mechanical, magnetic, and/or electrical features. In some embodiments, a sterile barrier may also be used between the surgical stapler 100 and the robotic arm 830. It should be appreciated that mounting of the surgical stapler 100 to the robotic arm places the surgical stapler 100 into communication and under the control of the control system 820 of the robotic manipulator 804, which is controlled by the control console 802 as discussed above.

The tool interface 900 includes a set of input pucks 910, which are manipulable to control functions of the surgical stapler 100 such as the positioning and firing of the end effector 110. Each input puck 910 is configured to mate with a corresponding puck driver 912 of the arm interface 902. To do so, each input puck 910 includes mating features 920 that are configured to mate with mating features 922 of the corresponding puck driver 912. In the illustrative embodiment, the mating features 920 of the input pucks 910 are embodied as tabs or protrusions that extend upwardly from the corresponding input puck 910, and the mating features 922 of the puck drivers 912 are embodied as recesses configured to receive the tabs/protrusions 920 of the input pucks 910. In other embodiments, however, the mating features 920 of the input pucks 910 may be embodied as recesses and the mating features 922 of the puck drivers 912 may be embodied as tabs/protrusions.

The arm interface 902 includes an electric motor 930 operatively coupled to each puck driver 912. Each electric motor 930 is communicatively coupled to and controlled by the control system 820. Actuation of a given electric motor 930 causes actuation of the associated puck driver 912, which causes actuation of the corresponding input puck 910. For example, rotation of an electric motor 930 causes rotation of the associated puck driver 912, which causes rotation of the corresponding input puck 910. In this way, the positioning and activation (e.g., "firing") of the end effector 110 of the surgical stapler 100 may be controlled via actuation of the puck drivers 912 and associated input pucks 910.

The tool interface 900 also includes a set of electrical connectors 940, which are configured to mate with corresponding electrical connectors 942 of the arm interface 902. The electrical connectors 940, 942 provide electrical communication between the surgical stapler 100 and the robotic manipulator 804. However, in other embodiments, the surgical stapler 100 and the robotic manipulator 804 may be configured to communicate in other ways, such as via wireless communications. In some embodiments, the tool interface 900 may also include a control system 950. In such embodiments, the control system 950 may be configured to control and/or monitor various operations the surgical stapler 100. The control system 950 may include various electrical components, circuits, and/or devices, such as a processor, a memory, and a communication subsystem to enable communications of the components of the control system 950, which are not illustrated in FIG. 9 for clarity.

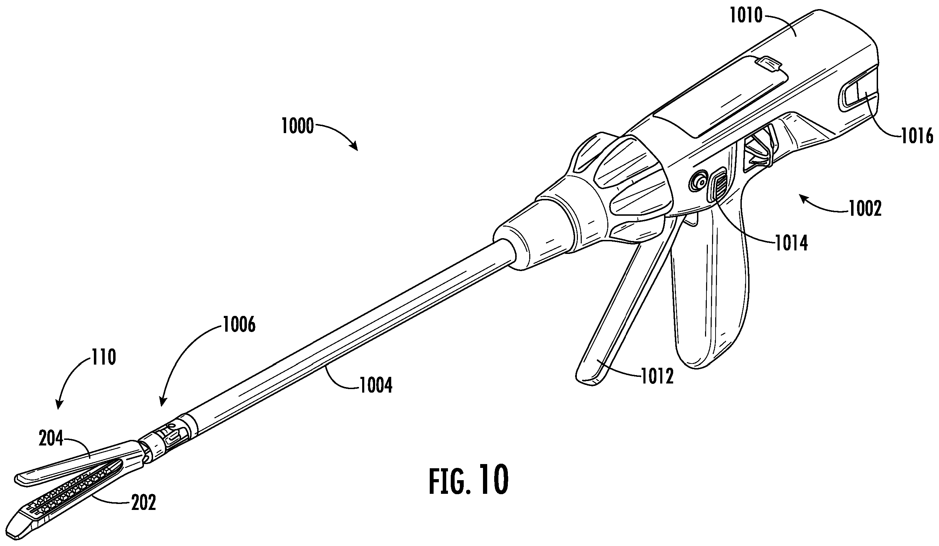

Although the illustrative surgical stapler 100 shown and described above in regard to FIGS. 1-9 is configured for use in a robotic surgical system, the features and concepts of the surgical stapler 100 described above and below may be applicable to hand-held surgical staplers and devices. For example, as shown in FIG. 10, the end effector 110 may be incorporated into a hand-held surgical stapler 1000. The surgical stapler 1000 includes a handle 1002 and an elongated shaft 1004 extending from the handle 1002. The distal end of the elongated shaft 1004 is coupled to the end effector 110 via an articulable joint 1006, which may be substantially similar to the articulable joint 106 described above.

The handle 1002 includes a handle housing 1010 and a trigger assembly 1012 movable connected to the handle housing 1010. The trigger assembly is operable by a surgeon to move the jaw assembly 200 of the end effector 110 from the open state to the closed state as discussed above. The handle 1002 also includes one or more activation triggers 1014 to, for example, initiate the firing of the end effector 110 when in the closed state. A replaceable and/or rechargeable battery pack 1016 is coupled to an end of the handle housing 1010 and provide power to the electrical components located within the handle housing 1010.

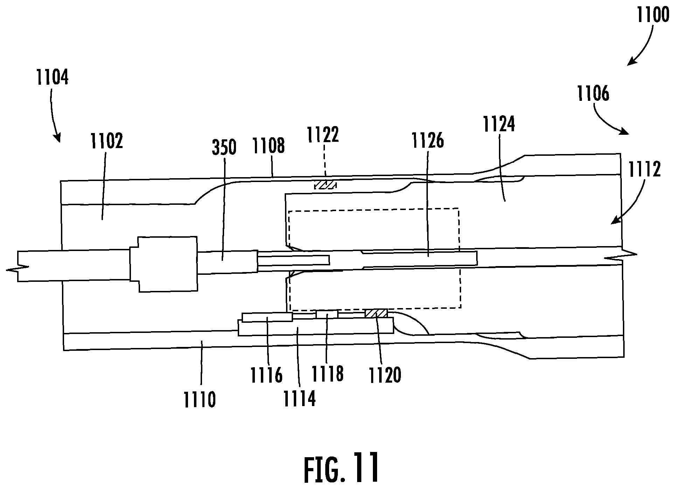

Referring now to FIG. 11, in an illustrative embodiment, the end effector 110 of the surgical stapler 100 and/or the hand-held surgical stapler 1000 may be embodied as an end effector 1100 having a cartridge jaw 1102 including an elongated body that extends from a proximal end 1104 to a distal end 1106. The illustrative end effector 1100 may be similar to an end effector 110 of the surgical stapler 100 and/or the hand-held surgical stapler 1000, and thus may include one or more components similar to the end effector 110 as described above.

As shown, the cartridge jaw 1102 includes a pair of parallel, opposing side walls 1108, 1110 that extend from the proximal end 1104 toward the distal end 1106. The side walls 1108, 1110 are connected by a floor and cooperate to define a channel 1112, which extends from the proximal end 1104 toward the distal end 1106.

The end effector 1100 further includes an electronics board 1114, which is illustratively coupled to an interior surface of the wall 1110, facing the channel 1112. The electronics board 1114 illustratively includes a controller 1116 and multiple magnetic field sensors 1118. The magnetic field sensors 1118 may be embodied as Hall effect sensors, three-dimensional Hall effect sensors, reed switches, or any other electronic sensor capable of measuring a magnitude and/or direction of magnetic field within the channel 1112 of the end effector 1100. Illustratively, a permanent magnet 1120 is also coupled to the electronics board 1116, within proximity of the magnetic field sensors 1118. However, other bodies or devices capable of generating a magnetic field may be used as an alternative to the permanent magnet(s) 1120.

As shown in FIG. 11 and as described above, a staple cartridge 1124 may be attached to the end effector 1100. When attached, the cartridge 1124 is illustratively inserted into the cartridge jaw 1102 of the end effector 1100, within the channel 1112. Each cartridge includes a sled 1126 that is moveable from a position toward the proximal end 1104 (as shown in FIG. 11) to another position toward the distal end 1108. During a firing operation of the surgical stapler 100 and/or the hand-held stapler 1000, the sled 1126 is moved by a drivetrain toward the distal end 1106. As the sled 1126 moves distally, the sled 1126 sequentially forces internal staple drivers upwards, which causes surgical staples to be ejected upward toward an anvil jaw of the end effector 1100 as discussed above. As the staples bear against the anvil jaw, they are bent into a shape that clamps the tissue that is currently between a top deck of the cartridge 1124 and the anvil jaw. Bending the staples into shape and clamping that tissue achieves hemostasis. After the sled 1126 has passed the tissue and hemostasis has been achieved, the knife 450 carried by the I-beam 350 (see FIG. 4) cuts (transects) the tissue. Accordingly, in order to assure safe operation of the surgical stapler 100 and/or the hand-held surgical stapler 1000, a firing operation must be performed only when a cartridge 1124 is correctly positioned within the channel 1112 and the sled 1126 of that cartridge 1124 is in the correct pre-firing position toward the proximal end 1104.

As described further below, the magnetic field sensors 1118 are capable of detecting or otherwise measuring magnetic field produced by the permanent magnet 1120 as well as any magnetic field distortion, interference, or other magnetic field changes caused by the presence of the sled 1126 within the channel 1112 of the end effector 1100. The controller 1116 is in communication with the magnetic field sensors 1118, and the controller 1116 may use magnetic field data determined by the magnetic field sensors 1118 to perform electronic lockout functions and/or perform other functionality of the surgical stapler 100 and/or the hand-held surgical stapler 1000.

Additionally or alternatively, although FIG. 11 illustrates the magnetic sensors 1118 and the permanent magnet 1120 as coupled to the same electronics board 1118 positioned on the same side wall 1110 of the end effector, it should be understood that in other embodiments those components may have a different position and/or arrangement. For example, in an embodiment, a permanent magnet 1122 may be coupled to an inner surface of the wall 1108, positioned across the channel 1112 from the magnetic field sensors 1118. In those embodiments, the permanent magnet 1122 may replace and/or supplement the permanent magnet 1120. Additionally or alternatively, in some embodiments the magnetic field sensors 1118 may be coupled to a different circuit board from the controller 1116 and/or the controller 1116 may be included in a different component of the surgical stapler 100 and/or the hand-held surgical stapler 1000, such as the housing 102 or the handle 1002, respectively. Additionally or alternatively, in some embodiments, the end effector 1100 may include one or more electromagnets, which may be used to supplement and/or substitute for the permanent magnet 1120. Continuing that example, one or more communication antennas (e.g., a near-field communication antenna) may be used as an electromagnet to generate a magnetic field.

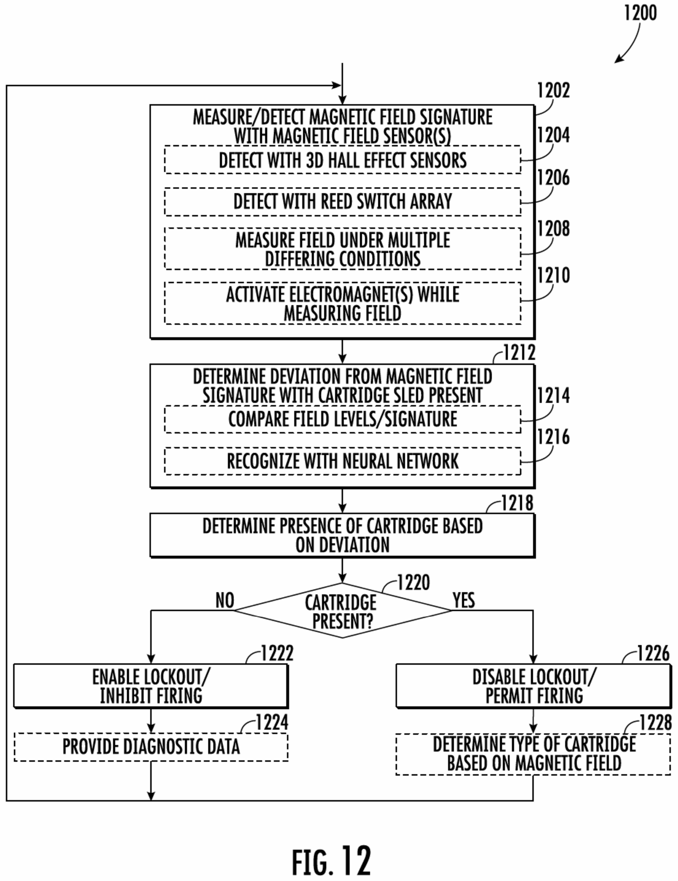

Referring now to FIG. 12, in use, the controller 1116 and/or other control element of the surgical stapler 100 and/or the hand-held surgical stapler 1000 (e.g., control system 950 or 820 discussed above) may perform a method 1200 for non-mechanical firing lockout using magnetic field detection. The method 1200 begins with block 1202, in which the controller 1116 measures and/or detects a magnetic field signature with one or more magnetic field sensors 1118 of the end effector 1100. The magnetic field signature may include one or more magnetic field magnitude and/or direction values, which may be measured by one or more magnetic field sensors. In some embodiments, in block 1204 the magnetic field signature may be measured with one or more three-dimensional Hall effect sensors 1118. For example, in those embodiments the magnetic field signature may include signed magnetic field magnitude values for each of three axes generated by a 3D Hall effect sensor 1118. The magnetic field signature may include one such magnetic field vector for each of the 3D Hall effect sensors 1118. In some embodiments, in block 1206, the magnetic field signature may be measured with a reed switch array that includes multiple reed switches 1118. For example, in those embodiments the magnetic field signature may include one or more magnitude values (e.g., binary values) generated by each reed switch in the array of reed switches.

In some embodiments, in block 1208, the controller 1116 may measure the magnetic field under multiple different conditions, and may include multiple magnetic field measurements in the magnetic field signature. In some embodiments, in block 1210, the controller 1116 may cause one or more electromagnets coupled to the end effector 1100 to be activated while measuring the magnetic field. In those embodiments, the magnetic field signature may include magnetic field measurements made while the electromagnet is active as well as measurements made while the electromagnet is not active. In some embodiments, the electromagnet may be embodied as a communication antenna such as a near-field communication (NFC) antenna coupled to the end effector 1100.

In block 1212, the controller 1116 determines a deviation between the measured magnetic field signature and a known magnetic field signature when a cartridge 1124 with sled 1126 is present in the end effector 1100 and in the ready for firing position. As described above, before firing, the cartridge 1124 must be attached to the cartridge jaw 1102 with the sled 1126 positioned toward the proximal end 1104 in the ready for firing position. The controller 1116 may use any appropriate technique to determine whether the measured magnetic field signature matches the known magnetic field signature when the sled 1126 is in the ready for firing position. In some embodiments, in block 1214, the controller 1116 may compare values of the measured magnetic field signature to values of the known magnetic field signature. For example, the controller 1116 may compare the magnetic field value for each axis in the measured magnetic field signature to a value to a corresponding axis in the known magnetic field signature and determine whether the measured value is within a predetermined threshold amount of the known value. In some embodiments, in block 1216, the controller 1116 may recognize whether the magnetic field signature matches the known field signature using a trained machine learning model such as an artificial neural network. It should be appreciated that, in other embodiments, other types of decision models (e.g., a heuristic model or other types of machine learning models) may be used.

In some embodiments, the known magnetic field signature may be developed by placing a representative of the cartridge 1124 with sled 1126 in the instrument 1100 in one or more known conditions (e.g., with the sled positioned proximal, positioned distal, positioned in a range therebetween, with the sled seated, with the sled lifted, positioned in a range therebetween, etc.) and recording the magnetic field signature. These signatures may be stored in the nonvolatile memory of the device and communicated to the controller 1116 during use, where the signatures may be accessed by a model to estimate the physical state of the jaw and cartridge.

In some embodiments, the sled 1126 of the cartridge 1124 may be placed in different mechanical configurations, and the resulting magnetic field signature is measured. The resulting magnetic field signature is compared to a standard magnetic field signature, and that offset may be stored in the cartridge. During use, that offset may be accessed from the cartridge 1124 and provided as an input to the model of the controller 1116 to more accurately determine cartridge state.

In some embodiments, one or more movable elements of the instrument 100 (e.g., jaw, wrist angle, knife, etc.) may be placed in known mechanical configurations, and the resulting magnetic field signature is captured and stored. The effect of the jaw position on the magnetic field signature may be used as an input to the model to better determine cartridge state.

In block 1218, the controller 1116 determines whether a cartridge 1124 is present in the end effector 1100 based on the measured magnetic field signature. For example, the controller 1116 may determine whether the determined deviation between the measured magnetic field signature and the known magnetic field signature exceeds a predetermined threshold, which may indicate that the cartridge 1124 is not present and/or that the sled 1126 is not in the correct position for firing. For example, if the sled 1126 is not in the ready for filing position (e.g., when the sled 1126 positioned toward the distal end 1106 after completion of a firing operation, when the cartridge 1124 is improperly installed in the end effector 1100, or in other circumstances when the sled 1126 is not ready for firing), then the measured magnetic field signature will differ from the known magnetic field signature with the sled 1126 in the correct position. As another example, the controller 1116 may recognize whether the cartridge 1124 is present based on output from a decision model, including a trained machine learning model such as an artificial neural network.

In block 1220, the controller 1116 checks whether the cartridge 1124 is present. If not, the method 1200 branches to block 1222, in which the controller 1222 enables a lockout or otherwise inhibits firing of the surgical stapler 100 and/or the hand-held surgical stapler 1000. The controller 1116 may use any appropriate technique to inhibit firing, for example by sending one or more control signals to components of the surgical stapler 100 and/or the hand-held surgical stapler 1000, disabling or deactivating one or more components of the surgical stapler 100 and/or the hand-held surgical stapler 1000, or otherwise preventing a firing operation while a cartridge 1124 is not present.

After enabling the lockout, in some embodiments in block 1224 the controller 1116 may provide diagnostic data, for example to one or more other components of the surgical stapler 100 and/or the hand-held surgical stapler 1000 and/or to a display device. For example, the controller 1116 may provide an indication that the cartridge 1124 is misaligned or otherwise incorrectly installed. Continuing that example, the controller 1116 may determine a degree or amount of misalignment based on the deviation between the measured magnetic field signature and the known magnetic field signature. In that example, a user may re-position the cartridge 1124, replace the cartridge 1124, or otherwise correct errors based on the indicated diagnostic data. After enabling the lockout, the method 1200 loops back to block 1202, in which the controller 1116 may continue to monitor the magnetic field signature.

Referring again to block 1220, if the controller 1116 determines that a cartridge 1124 is present, the method 1200 branches to block 1226, in which the controller 1116 disables a lockout or otherwise permits firing of the surgical stapler 100 and/or the hand-held surgical stapler 1000. The controller 1116 may use any appropriate technique to permit firing, for example by sending one or more control signals to components of the surgical stapler 100 and/or the hand-held surgical stapler 1000, enabling or activating one or more components of the surgical stapler 100 and/or the hand-held surgical stapler 1000, or otherwise allowing a firing operation while a cartridge 1124 is present. After disabling the lockout, in some embodiments in block 1228 the controller 1116 may determine a type of the cartridge 1124 that is present based on the measured magnetic field signature. For example, as described further below, in some embodiments different types of cartridges 1124 may include sleds 1126 with different features that generate respective different known magnetic field signatures. The controller 1116 may, for example, compare the measured magnetic field signature to a library of known magnetic field signatures for those sleds 1126 and determine the matching type of cartridge 1124. The controller 1116 may provide an identification of the type of the cartridge 1124 to another component of the surgical stapler 100 and/or the hand-held surgical stapler 1000 or otherwise control operation of the surgical stapler 100 and/or the hand-held surgical stapler 1000 based on the determined type of the cartridge 1124. After disabling the lockout, the method 1200 loops back to block 1202, in which the controller 1116 may continue to monitor the magnetic field signature.

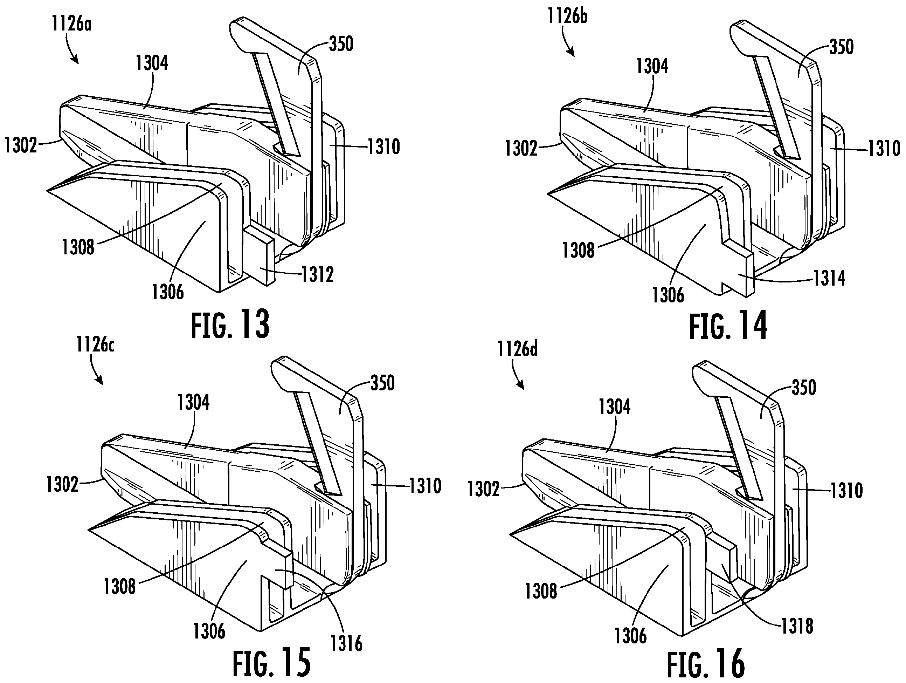

Referring now to FIGS. 13-16, multiple potential embodiments of sleds 1126 of the cartridge 1124 are shown. For example, a sled 1126a is shown in FIG. 13, a sled 1126b is shown in FIG. 14, a sled 1126c is shown in FIG. 15, and a sled 1126d is shown in FIG. 16. As described above, in some embodiments, each of the sleds 1126a-1126d may be used with different types of cartridges 1124 (e.g., cartridges with different sizes, different gauge staples, or otherwise different types of cartridges). Each sled 1126a-1126d includes a body 1302, which is formed from a magnetic material such as ferromagnetic metal. The body 1302 includes a central fin 1304 that extends upward from the body 1302, and multiple angled outer fins 1306, 1308, 1310 that extend upward from the body 1302. As shown, each of the angled outer fins 1306, 1308, 1310 includes an angled upper surface. As described above, when the sled 1126 moves from the pre-firing position at the proximal end 1104 toward the distal end 1108, those angled outer fins 1306, 1308, 1310 bear against internal drivers within the cartridge 1124, which move upward and eject surgical staples out of the cartridge 1124.

As shown, each of the sleds 1126a-1126d include fin extensions that extend proximally from one or more of the angled outer fins 1306, 1308, 1310. For example, the sled 1126a shown in FIG. 13 includes a fin extension 1312 extending proximally from the fin 1308. The sled 1126b shown in FIG. 14 includes a fin extension 1314 extending proximally 1314 from the fin 1306. The sled 1126c shown in FIG. 15 includes a fin extension 1316 extending proximally from the fin 1306. As shown, the fin extension 1316 is in a higher position relative to the body 1302 as compared to the fin extension 1314. The sled 1126d shown in FIG. 16 includes a fin extension 1318 extending proximally from the fin 1308. As shown, the fin extension 1318 is in a higher position relative to the body 1302 as compared to the fin extension 1312. Similar to or the same as the sled 1126, each of the fin extensions 1312, 1314, 1316, 1318 is formed from a magnetic material such as a ferromagnetic material. Accordingly, the magnetic field signature measured by the magnetic field sensors 1318 may differ based on the positions of the relative fin extensions 1312, 1314, 1316, 1318. Therefore, as described above, by detecting the fin extensions 1312, 1314, 1316, 1318, the controller 1116 may identify the various sleds 1126a, 1126b, 1126c, 1126d, respectively. Those sleds 1126a-1126d may be included in cartridges 1124 of different types, thereby allowing the controller 1116 to identify different cartridge types.

Continuing that example, in an embodiment the magnetic field sensors 1118 may include an array of magnetic field switches, which may be embodied as an array of reed switches arranged vertically along an inner surface of one of the walls 1108, 1110 of the cartridge jaw 1102. When one of the sleds 1126a-1126d is positioned in the channel in the ready-for-firing position, the corresponding fin extensions 1312, 1314, 1316, 1318 may cover or otherwise occlude one or more reed switches of the array of reed switches. When a reed switch 1118 is covered, then the magnetic field from the permanent magnet 1120 is blocked, warped, redirected, or otherwise modified, and is thus prevented from reaching the covered reed switch, and therefore that reed switch is not activated. Accordingly, based on the pattern of activated reed switches, the controller 1116 may determine whether a sled 1126 is present and, if so, which sled 1126 is present. For example, if all of the reed switches in the array are activated, this indicates that none of the reed switches are covered, and thus that no sled 1126 is present. In this situation, the controller 1116 may determine that no sled 1126 is present and enable lockout or otherwise inhibit firing. As another example, if only upper switch(es) in the reed array are activated, this indicates that lower switch(es) in the reed array are covered, and thus a sled 1126a or 1126b is present. As yet another example, if only lower switch(es) in the reed array are activated, this indicates that upper switch(es) in the reed array are covered, and thus a sled 1126c or 1126d is present. Of course, a different number and/or arrangement of fin extensions and/or magnetic field switches may be used. For example, in some embodiments, the fin extensions may have different vertical sizes and block different numbers of switches in the reed switch array. As another example, the various different fin extensions may be detected using other types of magnetic field sensors 1118 such as 3D Hall sensors.

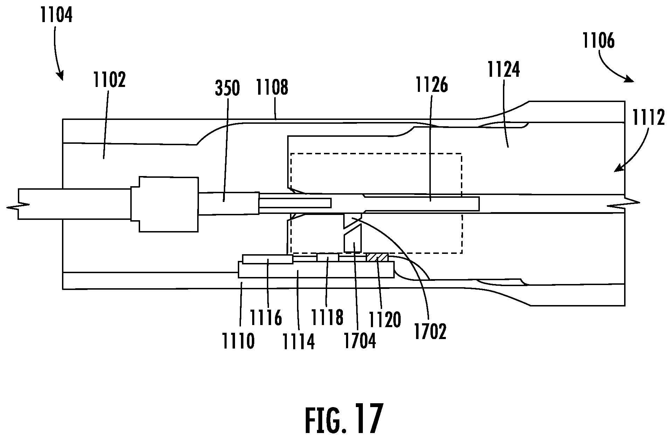

Referring now to FIG. 17, another embodiment of an end effector 1100 with magnetic field sensing is shown. The end effector 1100 of FIG. 17 includes similar components to the end effector shown in FIG. 11, the description of which is not repeated herein so as not to obscure the disclosure. Additionally, as shown in FIG. 17, the illustrative sled 1126 includes a lateral extension 1702 extending laterally toward the wall 1110 upon which the magnetic field sensors 1118 are mounted. The end effector 1100 further includes a magnetic driver 1704, which is formed from a magnetic material and is moveable from a position within to the channel 1112 to a position closer to the wall 1110, within range of the pairing of the magnetic field sensor 1118 and permanent magnet 1120. As shown, the lateral extension 1702 of the sled 1126 actuates the driver 1704, which moves the driver 1704 toward the magnetic field sensor 1118 and the permanent magnet 1120. This causes a change in the detected magnetic field signature, which may indicate presence of the sled 1126. The sled 1126 may actuate the driver 1704, for example, when installed in the cartridge jaw 1102, during a firing operation while the sled 1126 is moved from its pre-firing position toward the distal end 1108, or at another appropriate time.

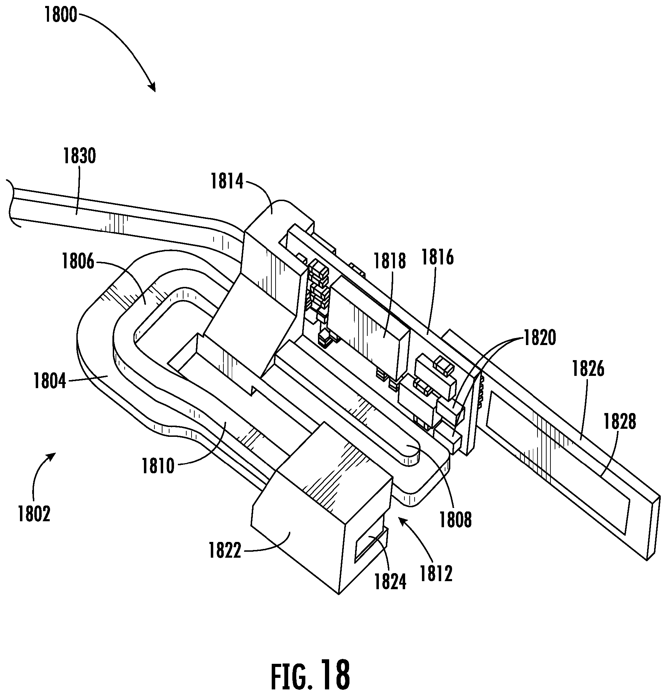

Referring now to FIGS. 18-20, an embodiment of an end effector 1100 includes an electronics system 1800 that may execute the method 1200. As shown in FIG. 18, the system 1800 includes a board support 1802, which may be formed from rigid plastic or another appropriate structural material. The board support 1802 includes a U-shaped frame 1804 that includes a bend 1806 and two arms 1808, 1810 extending distally from the bend 1806. A gap 1812 is defined between the two arms 1808, 1810.

The board support 1802 further includes a board holder 1814 extending upward from the arm 1808. The board holder 1814 includes a slot that receives a circuit board or other electronic component. Illustratively, the board holder 1814 receives a main board 1816 for the electronics subsystem 1800. The main board 1816 illustratively includes a processor or controller 1818 and one or more magnetic sensors 1820, such Hall effect sensors and/or an array of reed switches as described above. The main board 1816 may include additional electronic components in other embodiments.

The board support 1802 also includes a magnet holder 1822 that extends upward from the arm 1810. The magnet holder 1822 includes a slot that receives a permanent magnet 1824. As shown, the permanent magnet 1824 is positioned opposite a portion of the main board 1816, which illustratively includes the magnetic sensors 1820.

The electronics system 1800 further includes a near-field communication (NFC) board 1826 coupled to the main board 1816. The NFC board 1826 includes an antenna 1828, which may be used for RFID or other near-field communication functions. The NFC antenna 1828 may be used as an electromagnet as described above in connection with the method of FIG. 12.

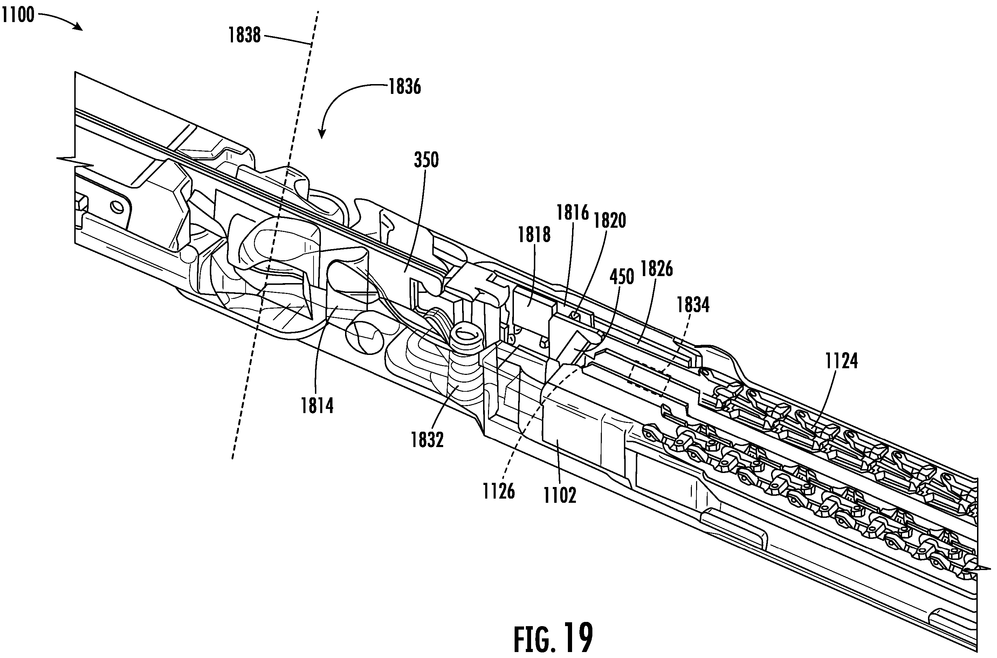

As shown in FIGS. 19-20, electronics system 1800 may be coupled to the cartridge jaw 1102 of the end effector 1100. As shown, when the electronics system 1800 is coupled to the end effector 1100, the board support 1802 is positioned toward the proximal end 1104 of the cartridge jaw 1102. The main board 1816 is positioned adjacent to the side wall 1108 of the cartridge jaw 1102. The permanent magnet 1824 is positioned on the opposite side of the channel 1112, opposing the magnetic field sensors 1820. The NFC board 1826 extends distally from the main board 1808 toward the distal end 1106, and is also positioned adjacent to the side wall 1108. The end effector 1100 further includes a spring bias lifter 1832, which is illustratively a helical spring, and is positioned proximal of the permanent magnet 1824.

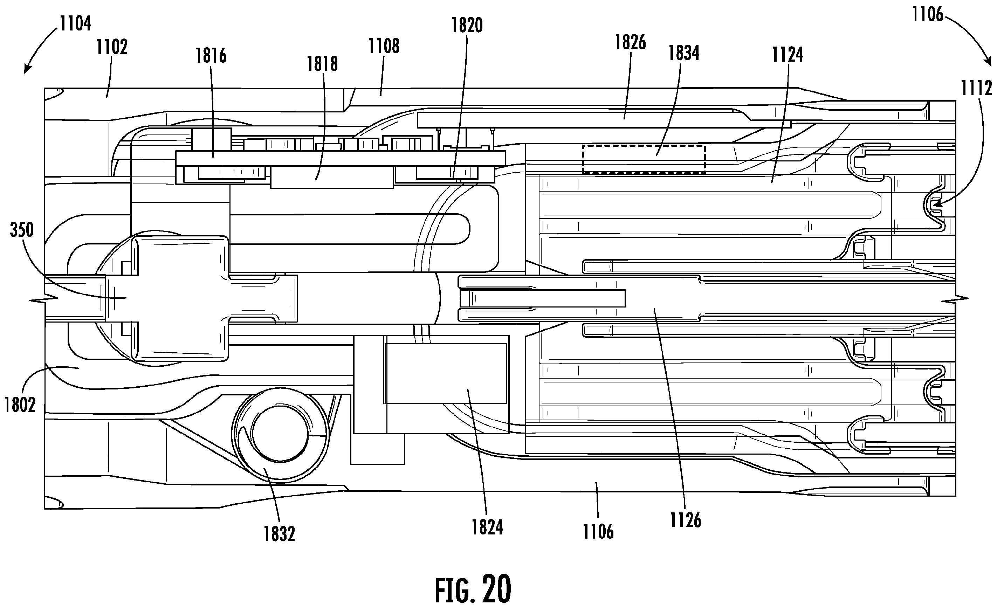

As shown in FIG. 20, when a cartridge 1124 is positioned in the channel 1112 of the jaw 1112, the sled 1126 is positioned between the permanent magnet 1824 and the magnetic sensors 1820. Accordingly, the controller 1818 may use the magnetic sensors 1820 to detect the presence of the sled 1126 by executing the method 1200 as described above in connection with FIG. 12.

Additionally or alternatively, as shown in FIGS. 19-20, the staple cartridge 1124 may include a radio frequency identification (RFID) tag 1834. When the cartridge is coupled to the cartridge jaw 1102 as shown in FIG. 16, the RFID tag 1834 is positioned adjacent to the antenna 1828 on the NFC board 1826. Accordingly, the electronics system 1800 may use the NFC board 1826 to sense the presence of the RFID tag 1834, which indicates that a cartridge has been attached to the cartridge jaw 1102 and/or may provide identifying information or other data related to the loaded cartridge.

While the disclosure has been illustrated and described in detail in the drawings and foregoing description, such an illustration and description is to be considered as illustrative and not restrictive in character, it being understood that only illustrative embodiments have been shown and described and that all changes and modifications that come within the spirit of the disclosure are desired to be protected.

There are a plurality of advantages of the present disclosure arising from the various features of the methods, apparatuses, and systems described herein. It will be noted that alternative embodiments of the methods, apparatuses, and systems of the present disclosure may not include all of the features described yet still benefit from at least some of the advantages of such features. Those of ordinary skill in the art may readily devise their own implementations of the methods, apparatuses, and systems that incorporate one or more of the features of the present invention and fall within the spirit and scope of the present disclosure as defined by the appended claims.

### EXAMPLES

Example 1 includes a method for controlling a surgical instrument, the method comprising measuring, by a controller coupled to the surgical instrument, a magnetic field signature with a magnetic field sensor coupled to an end effector of the surgical instrument, wherein a permanent magnet is coupled to the end effector; determining, by the controller, a deviation between the magnetic field signature and a first predetermined magnetic field signature, wherein the first predetermined magnetic field signature is associated with a cartridge present state of the surgical instrument, wherein the cartridge present state occurs when a cartridge is attached to the end effector and a sled of the cartridge is positioned at a home position within the end effector; determining, by the controller, whether the surgical instrument is in the cartridge present state based on the deviation; and inhibiting, by the controller, firing of the surgical instrument in response to determining that the surgical instrument is not in the cartridge present state.

Example 2 includes the subject matter of Example 1, and wherein the sled of the cartridge comprises a ferromagnetic material.

Example 3 includes the subject matter of any of Examples 1 and 2, and wherein measuring the magnetic field signature comprises measuring the magnetic field signature with a three-dimensional Hall effect sensor.

Example 4 includes the subject matter of any of Examples 1-3, and wherein measuring the magnetic field signature comprises measuring the magnetic field signature with a plurality of reed switches.

Example 5 includes the subject matter of any of Examples 1-4, and wherein measuring the magnetic field signature comprises: measuring a first magnetic field value with the magnetic field sensor; activating an electromagnet coupled to the end effector; and measuring a second magnetic field value with the magnetic field sensor while the electromagnet is activated, wherein the magnetic field signature comprises the first magnetic field value and the second magnetic field value.

Example 6 includes the subject matter of Example 5, and wherein the electromagnet comprises a near-field communication antenna of the end effector.

Example 7 includes the subject matter of any of Examples 1-6, and wherein determining the deviation between the magnetic field signature and the first predetermined magnetic field signature comprises comparing a first plurality of magnetic field values of the magnetic field signature to a corresponding second plurality of magnetic field values of the first predetermined magnetic field signature; and determining whether the surgical instrument is in the cartridge present state comprises determining whether any value of the first plurality of magnetic field values differs by more than a predetermined threshold amount from the corresponding value of the second plurality of magnetic field values.

Example 8 includes the subject matter of any of Examples 1-7, and wherein determining the deviation between the magnetic field signature and the first predetermined magnetic field signature comprises providing the magnetic field signature to a decision model; and determining whether the surgical instrument is in the cartridge present state comprises recognizing the cartridge present state with the decision model.

Example 9 includes the subject matter of any of Examples 1-8, and further comprising determining, by the controller, a type of cartridge coupled to the end effector based on the magnetic field signature.

Example 10 includes the subject matter of Example 9, and wherein determining the type of the cartridge comprises comparing the magnetic field signature to a library of predetermined magnetic field signatures.

Example 11 includes the subject matter of any of Examples 1-10, and further comprising providing, by the controller, diagnostic data based on the magnetic field signature in response to determining that the surgical instrument is not in the cartridge present state.

Example 12 includes the subject matter of any of Examples 1-11, and further comprising permitting, by the controller, firing of the surgical instrument in response to determining that the surgical instrument is in the cartridge present state.

Example 13 includes a surgical instrument comprising an end effector comprising an elongated jaw that extends to a distal end, wherein the jaw comprises a first side wall, a second side wall, and a floor coupled between the first side wall and the second side wall, wherein the first side wall, the second side wall, and the floor cooperate to define a channel extending to the distal end; a permanent magnet coupled to the elongated jaw; and a magnetic field sensor coupled to the elongated jaw and configured to measure a magnetic field signature associated with the permanent magnet.

Example 14 includes the subject matter of Example 13, and further comprising an elongated staple cartridge positioned in the channel of the jaw, wherein the staple cartridge comprises a sled moveable from a proximal position to a distal position; wherein the magnetic field signature associated with the permanent magnet changes when the sled moves between the proximal position and the distal position.

Example 15 includes the subject matter of Example 14, and wherein the sled comprises a ferromagnetic material.

Example 16 includes the subject matter of any of Examples 14 and 15, and wherein the sled comprises an outer fin that extends upward, the outer fin comprising a fin extension that extends proximally from the outer fin, wherein the fin extension redirects magnetic field from the permanent magnet when the sled is in the proximal position.

Example 17 includes the subject matter of Example 16, and wherein the magnetic field sensor comprises an array of a plurality of magnetic field sensors, and wherein the fin extension prevents the magnetic field from the permanent magnet from reaching a predetermined selection of the plurality of magnetic field sensors when the sled is in the proximal position.

Example 18 includes the subject matter of any of Examples 14-17, and wherein: the sled comprises a lateral extension that extends toward the magnetic field sensor; the end effector comprises a magnetic driver; and when the sled moves from the proximal position to the distal position, the lateral extension forces the magnetic driver toward the magnetic field sensor, whereby the magnetic field signature associated with the permanent magnet changes.

Example 19 includes the subject matter of any of Examples 13-18, and wherein the permanent magnet is coupled to the first side wall and the magnetic field sensor is coupled to the first side wall adjacent to the permanent magnet.

Example 20 includes the subject matter of any of Examples 13-19, and wherein the permanent magnet is coupled to the first side wall and the magnetic field sensor is coupled to the second side wall across the channel from the permanent magnet.

Example 21 includes the subject matter of any of Examples 13-20, and further comprising a near-field antenna coupled to the end effector, wherein the near-field antenna is configured to generate a magnetic field when energized; and a magnetic field sensor is further configured to measure the magnetic field generated by the near-field antenna when energized.

Example 22 includes the subject matter of any of Examples 13-21, and further comprising a board support coupled to the elongated jaw, wherein the board support comprises a first arm and a second arm coupled by a bend; and a circuit board coupled to the first arm of the board support, wherein the circuit board comprises the magnetic field sensor; wherein the permanent magnet is coupled to the second arm of the board support, and wherein the permanent magnet is positioned across the channel from the magnetic field sensor.

Example 23 includes or more non-transitory, computer-readable storage media comprising a plurality of instructions that in response to being executed result in a controller performing the method of any of Examples 1-12.

## Claims

1. A surgical instrument comprising:
an end effector (1100) comprising an elongated jaw (1102) that extends to a distal end (1106), wherein the jaw (1102) comprises a first side wall (1108), a second side wall (1110), and a floor coupled between the first side wall (1108) and the second side wall (1110), wherein the first side wall (1108), the second side wall (1110), and the floor cooperate to define a channel (1112) extending to the distal end (1106);
a permanent magnet (1120) coupled to the elongated jaw (1102); and
a magnetic field sensor (1118) coupled to the elongated jaw (1102) and configured to measure a magnetic field signature associated with the permanent magnet (1120).

2. The surgical instrument of claim 1, further comprising:
an elongated staple cartridge (1124) positioned in the channel (1112) of the jaw (1102), wherein the staple cartridge (1124) comprises a sled (1126) moveable from a proximal position to a distal position;
wherein the magnetic field signature associated with the permanent magnet (1120) changes when the sled (1126) moves between the proximal position and the distal position.

3. The surgical instrument of claim 2, wherein:
the sled (1126) comprises an outer fin (1306, 1308, 1310) that extends upward, the outer fin (1306, 1308, 1310) comprising a fin extension (1312, 1314, 1316, 1318) that extends proximally from the outer fin (1306, 1308, 1310), wherein the fin extension (1312, 1314, 1316, 1318) redirects magnetic field from the permanent magnet (1120) when the sled (1126) is in the proximal position; and
the magnetic field sensor (1118) comprises an array of a plurality of magnetic field sensors (1118), and wherein the fin extension (1312, 1314, 1316, 1318) prevents the magnetic field from the permanent magnet (1120) from reaching a predetermined selection of the plurality of magnetic field sensors (1118) when the sled (1126) is in the proximal position.

4. The surgical instrument of claim 2, wherein:
the sled (1126) comprises a lateral extension (1702) that extends toward the magnetic field sensor (1118);
the end effector (1100) comprises a magnetic driver (1704); and
when the sled (1126) moves from the proximal position to the distal position, the lateral extension (1702) forces the magnetic driver (1704) toward the magnetic field sensor (1118), whereby the magnetic field signature associated with the permanent magnet (1120) changes.

5. The surgical instrument of claim 1, further comprising:
a board support (1802) coupled to the elongated jaw (1102), wherein the board support (1802) comprises a first arm (1808) and a second arm (1810) coupled by a bend (1806); and
a circuit board (1816) coupled to the first arm (1808) of the board support (1802), wherein the circuit board (1816) comprises the magnetic field sensor (1118);
wherein the permanent magnet (1120) is coupled to the second arm (1810) of the board support (1802), and wherein the permanent magnet (1120) is positioned across the channel (1112) from the magnetic field sensor (1118).

6. A method for controlling a surgical instrument, the method comprising:
measuring (1202), by a controller (1116) coupled to the surgical instrument, a magnetic field signature with a magnetic field sensor (1118) coupled to an end effector (1100) of the surgical instrument, wherein a permanent magnet (1120) is coupled to the end effector (1100);
determining (1212), by the controller (1116), a deviation between the magnetic field signature and a first predetermined magnetic field signature, wherein the first predetermined magnetic field signature is associated with a cartridge present state of the surgical instrument, wherein the cartridge present state occurs when a cartridge (1124) is attached to the end effector (1100) and a sled (1126) of the cartridge (1124) is positioned at a home position within the end effector (1100);
determining (1218), by the controller (1116), whether the surgical instrument is in the cartridge present state based on the deviation; and
inhibiting (1222), by the controller (1116), firing of the surgical instrument in response to determining that the surgical instrument is not in the cartridge present state.

7. The method of claim 6, wherein measuring (1202) the magnetic field signature comprises measuring the magnetic field signature with a three-dimensional Hall effect sensor (1118).

8. The method of claim 6, wherein measuring (1202) the magnetic field signature comprises measuring the magnetic field signature with a plurality of reed switches (1118).

9. The method of claim 6, wherein measuring (1202) the magnetic field signature comprises:
measuring a first magnetic field value with the magnetic field sensor (1118);
activating an electromagnet coupled to the end effector (1100); and
measuring a second magnetic field value with the magnetic field sensor (1118) while the electromagnet is activated, wherein the magnetic field signature comprises the first magnetic field value and the second magnetic field value.

10. The method of claim 9, wherein the electromagnet comprises a near-field communication antenna (1828) of the end effector (1100).

11. The method of any one of claims 6-10, wherein:
determining (1212) the deviation between the magnetic field signature and the first predetermined magnetic field signature comprises comparing a first plurality of magnetic field values of the magnetic field signature to a corresponding second plurality of magnetic field values of the first predetermined magnetic field signature; and
determining (1218) whether the surgical instrument is in the cartridge present state comprises determining whether any value of the first plurality of magnetic field values differs by more than a predetermined threshold amount from the corresponding value of the second plurality of magnetic field values.

12. The method of any one of claims 6-10, wherein:
determining (1212) the deviation between the magnetic field signature and the first predetermined magnetic field signature comprises providing the magnetic field signature to a decision model; and
determining (1218) whether the surgical instrument is in the cartridge present state comprises recognizing the cartridge present state with the decision model.

13. The method of any one of claims 6-10, further comprising determining (1228), by the controller (1116), a type of cartridge (1124) coupled to the end effector (1100) based on the magnetic field signature.

14. The method of any one of claims 6-10, further comprising providing (1224), by the controller (1116), diagnostic data based on the magnetic field signature in response to determining that the surgical instrument is not in the cartridge present state.

15. One or more non-transitory, computer-readable storage media comprising a plurality of instructions that in response to being executed result in a controller (1116) performing the method of any of claims 6-14.
